# EUROPÄISCHE PATENTSCHRIFT

(11) **EP 2 033 611 B1**
(45) Veröffentlichungstag und Bekanntmachung des Hinweises auf die Patenterteilung: **11.04.2012**
(21) Anmeldenummer: 07450153.7
(22) Anmeldetag: 06.09.2007
(51) Int. Cl.: A61G 9/02, A61L 2/07

(54) **Methode und Vorrichtungen zur Dampfbeseitigung in Spül- und Desinfektionsautomaten für Steckbecken und Sanitätsartikel**
Method and devices for removing steam in rinsing and disinfection machines for bedpans and sanitary articles
Méthodes et dispositifs destinés à la suppression de la vapeur dans des automates de nettoyage et de désinfection pour des bassins de lit et des articles sanitaires

(43) Veröffentlichungstag der Anmeldung: 11.03.2009
(73) Patentinhaber: Clewat GmbH, 1010 Wien (AT)
(72) Erfinder: Collier, Jonathan, Cobham Surrey KT11 2DA (GB)
(74) Vertreter: KLIMENT & HENHAPEL

(56) Entgegenhaltungen:
- EP-A- 1 287 751
- WO-A-2005/037330
- DE-A1- 2 814 147

## Beschreibung

Vorliegende Erfindung betrifft einen Spül- und Desinfektionsautomaten für Steckbecken, Urinflaschen und ähnliche Sanitätsartikel, der insbesondere in Krankenhäusern und Pflegeheimen eingesetzt wird. Dieses Gerät desinfiziert das Spülgut mit Dampf, kühlt es nach der Desinfektion durch spezielle Vorrichtungen ab und beseitigt den Dampf aus der Spülkammer, damit dem Personal beim Öffnen des Spülgeräts keine heiße Dampfwolke entgegenschlägt. Die Methode der Dampfbeseitigung ist neu und Teil der Erfindung.

Darüber hinaus verfügt das Gerät entsprechend der Erfindung auch über geeignete Vorrichtungen, die die Stagnation von Wasser im Wasserkreislauf vermeiden.

Seit vielen Jahren gibt es Spülgeräte, die in Krankenhäusern und Pflegeheimen zur Reinigung von Sanitätsartikeln wie Steckbecken, Urinflaschen und Ähnlichem verwendet werden. Diese Geräte bestehen im Wesentlichen aus einer Spülkammer, in der die Sanitätsartikel starken Wasserstrahlen, eventuell unter Zusatz von Reinigungsmitteln, ausgesetzt werden, wodurch das Spülgut sorgfältig gereinigt wird. Die Spülkammer ist normalerweise direkt mit einem Abfluss in der Art eines WC-Abflusses verbunden, in den das am Spülgut haftende organische Material befördert wird.

Sowohl, was die Effizienz der Reinigung, als auch, was die Hygiene angeht, werden die Spülung und anschließende Nachspülung mit sehr heißem Wasser, wenn nicht sogar mit gesättigtem Dampf, durchgeführt. Als Folge davon sind die Spülkammer und das gereinigte Spülgut am Ende der Spülung sehr heiß, so dass dem Personal beim Öffnen der Spülkammertür zur Herausnahme des Spülguts eine unangenehme Dampfwolke entgegenschlägt.

Da sich in Krankenhäusern und Pflegeheimen häufig im selben Raum mehrere Geräte derselben Art und weitere Spülgeräte für andere Artikel befinden, bei denen insbesondere die Dampfsterilisation eingesetzt wird, darf nicht unterschätzt werden, dass der in die Luft abgegebene Dampf das Mikroklima stark verändern und zur Schimmelbildung an den Wänden führen kann.

Ein weiteres Problem bei dieser Geräteart besteht darin, dass im Wasserkreislauf, zu dem ein Speichertank, eine Spülpumpe, diverse Leitungen und eventuelle Ventile gehören, Restwasser stagniert. Diese Tatsache stellt an sich kein Problem dar, sofern die Intervalle zwischen den Spülungen nicht zu groß sind. Sollten zwischen einer Spülung und der nächsten 24, 36, 48 oder mehr Stunden vergehen, können sich Algen und

Pilze bilden, die sowohl an sich ein Problem darstellen als auch zu Fäulnis führen können.

Normalerweise stehen diese Geräte nicht über so lange Zeit still, aber es kann passieren, dass in einer Abteilung, in der mehrere dieser Geräte vorhanden sind, in einer Zeit geringerer Anwendungshäufigkeit vor allem einige bestimmte Geräte auf Kosten anderer verwendet werden, bei denen es bei tagelangem Betriebsstillstand zur Algen- und Pilzbildung kommen kann.

Das Problem der Dampfbeseitigung wird zumindest teilweise auf der Grundlage der Lehren aus dem Europa-Patent EP 0679406 B1 und dem Europa-Patentantrag EP 1 673 111 A1 gelöst, die Vorrichtungen für die Reinigung medizinischer Geräte, wie zahnärztliche Geräte, Steckbecken, Urinflaschen und Ähnliches, beschreiben, bei denen die Desinfektion mit Dampf erfolgt, der anschließend mit einem kalten Luftfluss, der auch das gereinigte, desinfizierte Spülgut abkühlt, in den Abfluss befördert wird.

Ungelöst bleibt jedoch das Problem der Beseitigung des im Wasserkreislauf stagnierenden Wassers.

Vorliegende Erfindung löst das Problem der Restdampfbeseitigung aus der Spülkammer völlig. Dazu werden nach Patentanspruch 1 und 2 eine Methode und ein Gerät zur Anwendung dieser Methode vorgeschlagen.

Durch eine bevorzugte Anwendungsform der Erfindung kann mit dem Gerät auch das Stagnieren von Wasser im Wasserkreislauf vermieden werden.

Die Erfindung betrifft also einen Spül- und Desinfektionsautomaten für Steckbecken und ähnliche Sanitätsartikel, der über Hauptvorrichtungen für die Abkühlung des Spülguts nach der Reinigung und die Beseitigung des Desinfektionsdampfes verfügt, damit beim Öffnen der Spülgerätetür keine heiße Dampfwolke austritt und dem Personal entgegenschlägt.

Die Erfindung wird durch Nebenvorrichtungen ergänzt, die das gesamte Wasser aus dem Wasserkreislauf beseitigen und so einer möglichen Stagnation vorbeugen.

Bei der Methode zur Abscheidung des in der Spülkammer vorhandenen Dampfes wird kalte Luft in die Spülkammer geblasen, so dass sie sich mit dem Dampf mischt, und das Dampf-Luft-Gemisch in eine Leitung befördert, in der es mit kaltem Wasser bespritzt wird, wodurch der Dampf kondensiert; dann werden das Spritzwasser und das aus der Dampfkondensation resultierende Wasser in den Abfluss befördert.

Die Hauptvorrichtungen, die die Abkühlung des Spülguts nach der Reinigung und die Beseitigung des Desinfektionsdampfes betreffen, bestehen aus einem Kompressor, der kalte Luft in die Spülkammer einfließen lässt, einer mit dem Abfluss verbundenen Leitung, in den der mit Luft gemischte Dampf befördert wird, und einem oder mehreren Zerstäubern, die das Gemisch besprühen, um den Dampf kondensieren zu lassen.

Im Unterschied zu den bekannten Geräten wird der Dampf zuerst kondensiert und dann zusammen mit dem die Kondensierung auslösenden Wasser über den Abfluss beseitigt, während die vom Dampf befreite Spülluft wieder an die Raumluft abgegeben werden kann.

Die Nebenvorrichtungen, die das gesamte Wasser aus dem Wasserkreislauf beseitigen, bestehen aus einer Leitung, die alle Stellen, an denen Wasser stagnieren kann, mit dem Abfluss verbindet.

Die Leitung, in der die Dampfkondensation erfolgt, ist nach oben auch an einen Wasserspeichertank angeschlossen. Der Anschluss erfolgt über eine Überlaufleitung, die den Abfluss überschüssigen Wassers gestattet und das Überlaufen an die Außenseite des Geräts verhindert.

Bei Einsatz eines mit der Erfindung übereinstimmenden Geräts kann das gereinigte, desinfizierte Spülgut abgekühlt, der Dampf in der Spülkammer abgeschieden und jegliches Stagnieren von Wasser im Wasserkreislauf vermieden werden, ohne dass ein Teil des Dampfes nach außen austritt. Daraus ergeben sich nicht zu vernachlässigende Vorteile für die Sauberkeit des Geräts und somit für die Hygiene. Das Problem der lästigen Dampfwolke beim Öffnen der Gerätetür wird völlig eliminiert.

Die Erfindung wird nun zur Veranschaulichung, ohne eine Beschränkung zu bedeuten, nach einer bevorzugten Anwendungsform und unter Bezugnahme auf die beiliegende Abbildung, in der das Funktionsschema eines Spül- und

Desinfektionsautomaten für Steckbecken und ähnliche Sanitätsartikel entsprechend der Erfindung gezeigt ist, beschrieben.

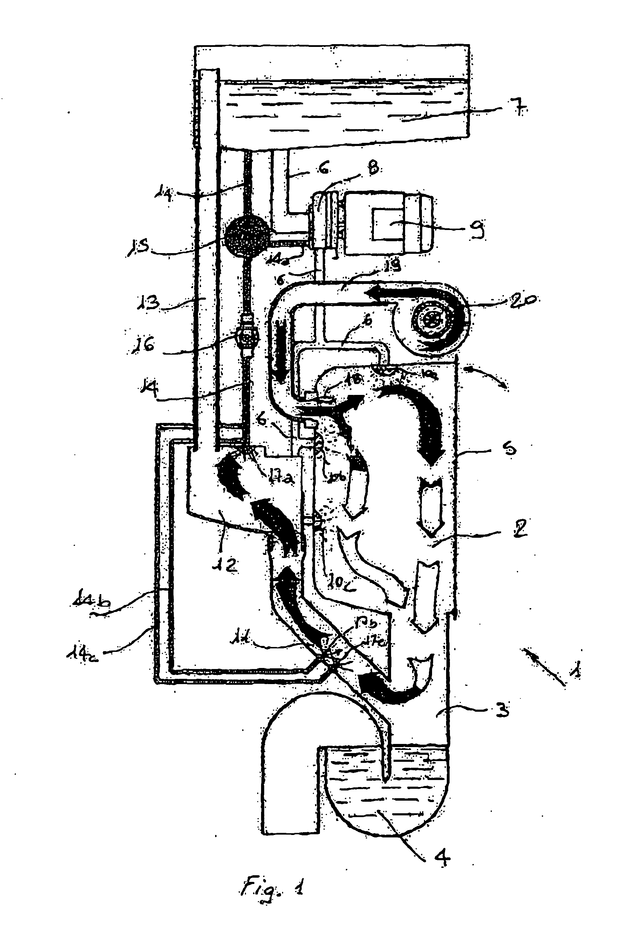

Mit Bezug auf Figur 1 wird mit 1 ein Spül- und Desinfektionsautomat für Steckbecken und ähnliche Sanitätsartikel entsprechend der Erfindung bezeichnet. Dieses Gerät 1 besteht aus einer Spülkammer 2, in die das (nicht dargestellte) Spülgut eingesetzt wird und die über eine Leitung 3 nach unten mit einer Abflussleitung 4 verbunden ist. An mehreren Punkten der Spülkammer 2, die über eine zu öffnende Tür 5 mit dem Außenraum verbunden ist, fließt eine vom Speichertank 7 des Spülwassers kommende Leitung 6 zu. Der Tank 7 ist mit dem Außenraum verbunden. Das Spülwasser wird von einer Pumpe 8 mit Motorantrieb 9 unter Druck gesetzt.

An den Punkten, an denen die Leitung 6 in die Spülkammer 2 eintritt, befinden sich Düsen 10a, 10b, 10c bekannter Art, die Wasserstrahlen zur Spülung und Nachspülung erzeugen.

Das Gerät verfügt über eine Vorrichtung bekannter Art (nicht dargestellt wegen der Einfachheit der Zeichnung), die das Wasser bis zur Erzeugung von Dampf erhitzt, um sowohl mit heißem Wasser zu spülen als auch mit Dampf zu desinfizieren. Diese Vorrichtung ist über ein Rückschlagventil mit der Leitung 6, die die Düsen 10a, 10b, 10c versorgt, verbunden und befördert so Wasser oder Dampf in die Spülkammer 2.

In der Leitung 3, die die Spülkammer 2 mit der Abflussleitung 4 verbindet, fließt eine schräge Leitung 11 zusammen, die nach oben mit einem Becken 12 verbunden ist, in dem eine vom Speichertank 7 des Spülwassers kommende Überlaufleitung 13 und eine weitere ebenfalls vom Tank 7 kommende, aber das Wasser aus dem unteren Teil des Tanks entnehmende Leitung 14 zusammenfließen.

In die Leitung 14 sind eine Pumpe 15 und ein gesteuertes Ventil, beispielsweise ein Magnetventil 16, eingesetzt. Darüber hinaus ist die Leitung 14 über einen Leitungszweig 14a mit der Spülpumpe 8 verbunden. Im Genaueren ist sie an den niedrigsten Punkt der Pumpe 8 angeschlossen. An der Stelle, an der die Leitung 14 in das Becken 12 eintritt, ist vorzugsweise eine Zerstäuberdüse 17a angebracht, während die anderen beiden Zerstäuberdüsen 17b, 17c an einem Punkt der Leitung 11 angebracht sind und von Zweigen 14b, 14c der Leitung 14 versorgt werden, die vom unteren Bereich der Leitung 14 vor der Zerstäuberdüse 17a ausgehen.

An einem Punkt der Spülkammer 2 wird über ein Rückschlagventil 18 eine Leitung 19 eingesetzt, die von einem Kompressor 20 erzeugte Druckluft in die Spülkammer 2 einfließen lässt (die Luft wird dem Außenraum entnommen und vorzugsweise gefiltert).

Die Spülung des in die Spülkammer 2 des Geräts 1 entsprechend der Erfindung eingesetzten Spülguts erfolgt durch die Wirkung von unter Druck stehenden Wasserstrahlen, die aus den über die Leitung 6 mit Wasser aus dem Tank 7 versorgten Düsen 10a, 10b, 10c austreten und von der Spülpumpe 8 mit Motorantrieb 9 unter Druck gesetzt werden.

Nach der Spülung, die vorzugsweise mit heißem Wasser und Spülmittel erfolgt, folgt die Desinfektion mit gesättigtem Dampf. Nach Abschluss der Desinfektionsphase befindet sich in der Spülkammer 2 sehr heißer Dampf in großer Menge. Darüber hinaus ist auch das in der Spülkammer 2 befindliche Spülgut sehr heiß. Öffnete das Personal in diesem Moment die Tür 5, schlüge ihm heißer Dampf entgegen. In dieser Phase besteht eine konkrete Verletzungsgefahr.

Um dieses Problem zu umgehen, werden am Ende der Dampfdesinfektionsphase gleichzeitig der Kompressor 20 und die Pumpe 15 betätigt, während das Magnetventil 16 geöffnet wird, um den Wassereinfluss in die Leitung 14 und somit durch den Austritt aus den Zerstäuberdüsen 17a, 17b, 17c die Zerstäubung des Wassers im Becken 12 und in der Leitung 11 zu gestatten.

Die aus dem Kompressor 20 kommende Luft läuft durch die Leitung 19 und tritt über das Rückschlagventil 18 in die Spülkammer 2 ein.

Dadurch, dass die noch kalte Luft (gekennzeichnet mit den dunklen Pfeilen) in der Spülkammer 2 mit dem heißen Spülgut in Berührung kommt und sich mit dem Dampf mischt, erwärmt sie sich (weiße Pfeile) und fließt dann in die Leitung 11 und in das Becken 12 ab, wo sie durch die Wirkung des von den Düsen 17a, 17b, 17c zerstäubten Wassers abkühlt (dunkle Pfeile).

Die abgekühlte Luft steigt über das Becken 12 auf und wird dann an die Außenluft abgegeben.

Gleichzeitig mischt sich das von den Düsen 17a, 17b, 17c zerstäubte Wasser mit dem Wasser des kondensierten Dampfes und fließt über die Leitung 11 in die Abflussleitung 3, von der aus es eliminiert wird.

Wird weiter Luft über einen ausreichend langen Zeitraum eingeblasen, erzielt man neben der Dampfbeseitigung aus der Spülkammer 2 die Abkühlung des desinfizierten Spülguts, so dass es vom Personal leichter entnommen werden kann.

Die Leitung 14 wird im untersten Teil des Tanks 7 eingesetzt, so dass der Tank über diese Leitung komplett geleert werden kann. Über diese Leitung kann auch die Spülpumpe 8 komplett geleert werden, da sie mit der Pumpe über ihren Zweig 14a verbunden ist, der im untersten Teil der Pumpe 8 eingesetzt wird.

Wenn der Wasserkreislauf vor einem längeren Betriebsstillstand vollständig geleert werden soll, ist es ausreichend, das Magnetventil 16 geöffnet zu lassen, damit der Tank 7 und die Spülpumpe 8 sich in das Becken 12 entleeren und das Wasser von dort über die Leitung 11 in die Abflussleitung 4 abfließt.

Wie man aus vorstehender Beschreibung ersehen kann, ergibt sich bei Verwendung des Geräts 1 entsprechend der Erfindung folgendes Ergebnis: die Beseitigung des Dampfes, die Abkühlung des gereinigten, desinfizierten Spülguts sowie die vollständige Eliminierung des Wassers aus dem Wasserkreislauf bei Betriebsstillstand des Geräts.

Die Erfindung wurde zur Veranschaulichung, ohne eine Beschränkung zu bedeuten, nach einer bevorzugten Anwendungsform beschrieben. Der Fachtechniker kann andere Anwendungsformen der Erfindung ableiten, die alle in den Schutzbereich folgender Ansprüche fallen.

## Patentansprüche

1. Methode zur Abscheidung des in einer Spülkammer (2) von Spül- und Desinfektionsautomaten (1) für Steckbecken und ähnliche Sanitätsartikel nach der Desinfektion vorhandenen Dampfes, **gekennzeichnet durch** folgende Phasen:
- Einblasen von kalter Luft in das Innere der Spülkammer (2), in der sich die Luft mit dem Dampf mischt,
- Beförderung des Luft-Dampf-Gemischs in eine erste Leitung (11),
- Kondensieren des Dampfes in der ersten Leitung (11) durch Besprühen mit kaltem Wasser
- Abführen des Spritzwassers und des Kondensationswassers in eine Abflussleitung (4), wobei ein die Spülkammer (2) mit der Abflussleitung (4) verbindender Leitungsabschnitt (3) vorgesehen ist und die erste Leitung (11) von diesem Leitungsabschnitt (3) abzweigt bzw. in diesen einmündet,
- Abführen der Luft an einen Außenraum.

2. Spül- und Desinfektionsautomat (1) für Steckbecken und ähnliche Sanitätsartikel, umfassend
- eine Spülkammer (2), die von einer Spülpumpe (8) mit unter Druck stehendem Wasser bzw. Desinfektionsdampf und von einem Kompressor (20) mit kalter Luft zwecks Abkühlung des in der Spülkammer (2) befindlichen Spülguts nach dessen Reinigung und Desinfektion und zur Beseitigung von Desinfektionsdampf speisbar ist, wobei sich die kalte Luft mit dem Dampf mischt,
- eine an die Spülkammer (2) angeschlossene erste Leitung (11), die nach oben mit einem Außenraum in Verbindung steht, um vom Kompressor (20) eingeblasene Luft nach Abscheidung vom Dampf an die Aussenluft abzugeben, und nach unten mit einer Abflussleitung (4) in Verbindung steht,
- in der ersten Leitung (11) vorgesehene Kaltwasserdüsen (17b, 17c), deren Strahlen auf den aus der Spülkammer (2) beseitigten Dampf gerichtet sind, um ihn zu kondensieren,
- wobei die Abwasserleitung (4) so ausgebildet ist, dass das mit den Düsen (17b, 17c) versprühte Wasser und das Kondensationswasser des Dampfes und das in die Spülkammer (2) gespeiste Wasser in die Abwasserleitung (4) fließt,
- wobei ein die Spülkammer (2) mit der Abflussleitung (4) verbindender Leitungsabschnitt (3) vorgesehen ist und die Kaltwasserdüsen (17b, 17c) aufweisende und zur Aufnahme der mit Dampf vermischten Luft dienende erste Leitung (11) mit der Abflussleitung (4) in Verbindung steht, indem sie von diesem Leitungsabschnitt (3) abzweigt bzw. in diesen einmündet.

3. Spül- und Desinfektionsautomat nach Anspruch 2, **dadurch gekennzeichnet, dass** die aus dem Kompressor (20) kommende kalte Luft über ein Rückschlagventil (18) in die Spülkammer (2) einfließt.

4. Spül- und Desinfektionsautomat nach Anspruch 2, **dadurch gekennzeichnet, dass** die Kaltwasserdüsen (17b, 17c) über eine weitere Leitung (14, 14b 14c) mit Wasser versorgt werden, das von einer Pumpe (15) unter Druck gesetzt wird.

5. Spül- und Desinfektionsautomat nach Anspruch 4, **dadurch gekennzeichnet, dass** die weitere Leitung (14, 14b, 14c) von einem Speichertank (7) versorgt wird.

6. Spül- und Desinfektionsautomat nach Anspruch 5, **dadurch gekennzeichnet, dass** eine in die erste Leitung (11) mündende, im Speichertank (7) endende Überlaufleitung (13) vorgesehen ist.

7. Spül- und Desinfektionsautomat nach einem der Ansprüche 2 bis 6, **dadurch gekennzeichnet, dass** Nebenvorrichtungen vorgesehen sind, die das gesamte Wasser aus dem Wasserkreislauf entfernen und eine mögliche Stagnierung vermeiden.

8. Spül- und Desinfektionsautomat nach Anspruch 7, **dadurch gekennzeichnet, dass** als Bestandteil der Nebenvorrichtungen eine die Versorgungsleitungen (14a,14b,14c) aus dem Speichertank (7) speisende weitere Leitung (14) vorgesehen ist, die mit einem gesteuerten Ventil (16) ausgestattet ist, das eine unbeabsichtigte Leerung des Wasserkreislaufs verhindert.

9. Spül- und Desinfektionsautomat nach Anspruch 10, **dadurch gekennzeichnet, dass** an der weiteren Leitung (14) ein weiterer Zweig (14a) vorgesehen ist, der die Spülpumpe (8) mit der weiteren Leitung (14) verbindet.

## Claims

1. A method for removing steam present after disinfection in a rinsing chamber (2) of automatic rinsing and disinfection machines (1) for bedpans and similar sanitary articles, **characterized by** the following phases:
- injection of cold air into the interior of the rinsing chamber (2) in which the air mixes with the steam;
- conveying the air-steam mixture into a first line (11);
- condensing the steam in the first line (11) by spraying with cold water;
- discharging the splashwater and the condensation water to a drain pipe (4), with a line section (3) being provided which connects the rinsing chamber (2) with the drain pipe (4), and the first line (11) branches off from said line section (3) or opens into the same;
- discharging of the air to an external space.

2. An automatic rinsing and disinfection machine (1) for bedpans and similar sanitary articles, comprising
- a rinsing chamber (2) which can be supplied by a rinsing pump (8) with pressurized water or disinfection steam and by a compressor (20) with cold air for the purpose of cooling the rinsing materials disposed in the rinsing chamber (2) after their cleaning and disinfection and for removing disinfection steam, with the cold air mixing with the steam;
- a first line (11) which is connected to the rinsing chamber (2) and which is upwardly in connection with an external space in order to emit air injected by the compressor (20) after separation from steam to external air, and downwardly in connection with a drain pipe (4);
- cold-water nozzles (17b, 17c) which are provided in the first line (11) and the jets of which are directed against the steam removed from the rinsing chamber (2) in order to condensate said steam;
- with the drain pipe (4) being arranged in such a way that the water sprayed with the nozzles (17b, 17c) and the condensation water of the steam and the water supplied to the rinsing chamber (2) flow into the drain pipe (4);
- with a line section (3) being provided which connects the rinsing chamber (2) with the drain pipe (4) and with the first line (11) which comprises cold-water nozzles (17b, 17c) and is used for accommodating the air mixed with the steam being in connection with the drain pipe (4) in that it branches off from said line section (3) or opens into the same.

3. An automatic rinsing and disinfection machine according to claim 2, **characterized in that** the cold air coming from the compressor (20) flows into the rinsing chamber (2) via a non-return valve (18).

4. An automatic rinsing and disinfection machine according to claim 2, **characterized in that** the cold-water nozzles (17b, 17c) are supplied via a further line (14, 14b, 14c) with water which is pressurized by a pump (15).

5. An automatic rinsing and disinfection machine according to claim 4, **characterized in that** the further line (14, 14b, 14c) is supplied by a storage tank (7).

6. An automatic rinsing and disinfection machine according to claim 5, **characterized in that** an overflow line (13) is provided which opens into the first line (11) and ends in the storage tank (7).

7. An automatic rinsing and disinfection machine according to one of the claims 2 to 6, **characterized in that** auxiliary apparatuses are provided which remove the entire water from the water cycle and prevent potential stagnation.

8. An automatic rinsing and disinfection machine according to claim 7, **characterized in that** a further line (14) is provided as a component of the auxiliary apparatuses, said further line supplying the supply lines (14a, 14b, 14c) from the storage tank (7) and being provided with a controlled valve (16) which prevents any inadvertent draining of the water cycle.

9. An automatic rinsing and disinfection machine according to claim 10, **characterized in that** a further branch (14a) is provided on the further line (14) which connects the rinsing pump (8) with the further line (14).

## Revendications

1. Procédé pour séparer la vapeur présente après la désinfection dans une chambre de lavage (2) d'appareils automatiques de lavage et de désinfection (1) pour des bassins hygiéniques et articles sanitaires similaires, **caractérisé en ce qu'**il comprend les phases suivantes :
- injection d'air froid à l'intérieur de la chambre de lavage (2), dans laquelle l'air se mélange à la vapeur ;
- transport du mélange d'air et de vapeur dans une première conduite (11) ;
- condensation de la vapeur dans la première conduite (11) par arrosage avec de l'eau froide ;
- évacuation de l'eau d'arrosage et de l'eau de condensation dans une conduite d'écoulement (4), un tronçon de conduite (3) reliant la chambre de lavage (2) à la conduite d'écoulement (4) étant prévu et la première conduite (11) bifurquant à partir de ce tronçon de conduite (3) ou débouchant dans celui-ci ;
- évacuation de l'air vers un espace extérieur.

2. Appareil automatique de lavage et de désinfection (1) pour des bassins hygiéniques et articles sanitaires similaires, comprenant :
- une chambre de lavage (2) qui peut être alimentée par une pompe d'injection d'eau (8) en eau sous pression ou en vapeur de désinfection et par un compresseur (20) en air froid en vue du refroidissement des articles à laver se trouvant dans la chambre de lavage (2) après leur nettoyage et leur désinfection et en vue de l'élimination de la vapeur de désinfection, l'air froid se mélangeant à la vapeur ;
- une première conduite (11) raccordée à la chambre de lavage (2), qui communique vers le haut avec un espace extérieur afin de libérer l'air injecté par le compresseur (20) dans l'air extérieur après la séparation de la vapeur, et vers le bas avec une conduite d'écoulement (4) ;
- des buses d'eau froide (17b, 17c) prévues dans la première conduite (11), dont les jets sont dirigés vers la vapeur extraite de la chambre de lavage (2) afin de condenser celle-ci ;
- dans lequel la conduite d'évacuation (4) est conformée de telle manière que l'eau injectée avec les buses (17b, 17c) et l'eau de condensation de la vapeur et l'eau amenée dans la chambre de lavage (2) s'écoulent dans la conduite d'évacuation (4) ;
- dans lequel est il est prévu un tronçon de conduite (3) reliant la chambre de lavage (2) à la conduite d'écoulement (4) et la première conduite (11) présentant les buses d'eau froide (17b, 17c) et servant à recevoir l'air mélangé à de la vapeur communique avec la conduite d'écoulement (4) en bifurquant à partir de ce tronçon de conduite (3) ou en débouchant dans celui-ci.

3. Appareil automatique de lavage et de désinfection selon la revendication 2, **caractérisé en ce que** l'air froid venant du compresseur (20) entre dans la chambre de lavage (2) en passant par une soupape antiretour (18).

4. Appareil automatique de lavage et de désinfection selon la revendication 2, **caractérisé en ce que** les buses d'eau froide (17b, 17c) sont alimentées par une autre conduite (14, 14b, 14c) en eau qui est pressurisée par une pompe (15).

5. Appareil automatique de lavage et de désinfection selon la revendication 4, **caractérisé en ce que** l'autre conduite (14, 14b, 14c) est alimentée par un réservoir d'accumulation (7).

6. Appareil automatique de lavage et de désinfection selon la revendication 5, **caractérisé en ce qu'**il est prévu une conduite de trop-plein (13) débouchant dans la première conduite (11) et aboutissant au réservoir d'accumulation (7).

7. Appareil automatique de lavage et de désinfection selon l'une des revendications 2 à 6, **caractérisé en ce que** sont prévus des dispositifs secondaires qui retirent toute l'eau du circuit d'eau et évitent une éventuelle stagnation.

8. Appareil automatique de lavage et de désinfection selon la revendication 7, **caractérisé en ce qu'**il est prévu comme composant des dispositifs secondaires une autre conduite (14) qui alimente les conduites d'alimentation (14a, 14b, 14c) à partir du réservoir d'alimentation (7) et qui est équipée d'une vanne régulée (16) empêchant la vidange involontaire du circuit d'eau.

9. Appareil automatique de lavage et de désinfection selon la revendication 10, **caractérisé en ce qu'**il est prévu sur l'autre conduite (14) une autre branche (14a) qui relie la pompe d'injection d'eau (8) à l'autre conduite (14).
